# EUROPEAN PATENT APPLICATION

(11) **EP 1 275 763 A1**
(43) Date of publication of application: **15.01.2003**
(21) Application number: 01904324.9
(22) Date of filing: 07.02.2001
(51) Int. Cl.: D04H 1/42, D04H 3/16, A41B 13/00, A61F 5/44, A61F 13/15

(54) **NONWOVEN FABRIC, PROCESS FOR PRODUCING THE SAME, SANITARY MATERIAL, AND SANITARY SUPPLY**

(30) Priority: 10.02.2000 JP 2000033191
(71) Applicant: Idemitsu Unitech Co., Ltd., Tokyo 112-0002 (JP)
(72) Inventor: Ishikawa, Masahide, Sanbu-gun Chiba 283-0101 (JP)
(74) Representative: Hucker, Charlotte Jane
(86) International application number: JP0100834
(87) International publication number: WO01059193

(57) **Abstract**

Polyolefin resin-based non-woven fabric characterized by containing a fatty acid amide compound, and by having a static friction coefficient of 0.1 to 0.4. Production of such non-woven fabric is achieved by subjecting, for example, non-woven fabric containing 0.05 to 1 wt.% erucamide to an aging treatment at 30 to 60°c for 5 to 50 hours.

## Description

### Field of the Invention

The present invention relates to polyolefin resin-based non-woven fabric and a method for the production thereof, particularly to fabric suitable as a material of sanitary articles such as disposable diapers because of its excellent hand and touch feeling.

### Description of the Related Art

The filament non-woven fabric such as spunbonded non-woven fabric has been widely used in many applications because it has excellent mechanical properties such as high tensile strength and air permeability, as well as ensures the efficient production of fabric based on continuously spun fibers. The thermoplastic resin which has been mainly used as a material of such filament non-woven fabric includes polyamide resins and polyester resins because they give excellent fiber characteristics, and are easily melt into a fiber easily yielding to spinning (spinning easiness). However, currently, an increasingly larger amount of polyolefin resins such as polypropylene, polyethylene, etc., or the resins, which have been used, in general applications, come to be used as a material of the fabric in question.

To see how a polyolefin-based resin can serve as a material of non-woven fabric, take, for example, a polypropylene-based resin. Even if the polypropylene resin results from a single propylene monomer, the resulting homopolymers may have different crystallizing activities. Or, if propylene is polymerized together with ethylene or butene-1, the resulting copolymers will have different properties, for example, in the melting point, mechanical strength, elastic modulus, etc. Take non-woven fabric made of such polypropylene resins. If it is made of a resin having a high crystallizing activity, it will be excellent in spin easiness, but poor in softness and problematic in hand. On the contrary, if a polypropylene resin having a low crystallizing activity and melting point is used as a material of the non-woven fabric, the fiber of the fabric will be very resistant to spinning, because it shows a high friction against adjacent fibers or metals, although it will be excellent in softness.

If a polypropylene-based resin of which the isotactic pentad fraction (which serves as an index representing the crystallizing activity of the resin) is about 90 mol %, or a resin most often used as a material of non-woven fabric, is employed, the fiber will be comparatively good in spin easiness but the fabric based thereon will not always have a sufficient hand and touch feel to be effectively applied for the production of sanitary articles such as disposable diapers, sanitary napkins, incontinence pads, etc.

To cope with these problems, the Japanese Patent Laid-Open No. 10-88459 proposes filament non-woven fabric which is made of a thermally bonded filament bicomponent fiber: the bicomponent fiber is obtained by combining a first component having at least one low melting point or one low softening point selected from olefin double copolymers or olefin triple copolymers, and a second component composed of a crystalliable thermoplastic resin, a hydrocarbon lubricant being added at least to the first component, and the combination of the two components occurring in such a way as to make the content of the hydrocarbon lubricant in the bicomponent fiber to be 2 - 20 wt.%.

This proposal employs, as a polypropylene resin, a propylene-based random copolymer, which has a low melting point and is soft, to enhance the softness and touch feel of the resulting fabric, and tries to compensate for the degraded spin easiness characteristic with such a copolymer, by adding a hydrocarbon lubricant thereto. In the material of this fabric, however, the features characteristic with a propylene-based homopolymer such as high thermal resistance, high mechanical strength, etc are absent, and a comparatively large amount of a hydrocarbon compound with a low molecular weight and low melting point must be added to the propylene-based random copolymer. The coexistence of such a hydrocarbon lubricant may prove to be problematic: the heat seal and adhesiveness of the non-woven fabric will be degraded as a result of the brittleness introduced by the lubricant.

The Japanese Patent Laid-Open No. 8-13238 discloses a polypropylene resin composition for fiber production which is obtained by combining 100 parts by weight of a crystallizable polypropylene resin of which the ratio (Mw/Mn) of the weight average molecular weight (Mw) against the number average molecular weight (Mn) is 2 to 15, and of which the fraction of isotactic pentads is 96% or higher, with 0.01 to 1 part by weight of a fatty acid amide compound added.

This patent publication introduces a highly crystallizable polypropylene resin which ensures the production of a highly strong fiber whose drawing is increased 6 times or more than that of the common polypropylene resin-based fiber, with a lubricant added to enhance the drawing and luster of the fiber as well as to compensate for the otherwise degraded drawing of the fiber which might result from fuzz which otherwise might develop during the extension of the fiber. Namely, the resin has a crystallizing activity different from that of the resin commonly used as a material of a polypropylene resin-based non-woven fabric; but the fabric based on the resin does not always give a good softness, hand and touch feel. Indeed, although the patent publication mentions that the fabric based on the resin is suitably used as a material of non-woven fabric for industrial use, it gives no mention regarding its applicability to the production of sanitary materials, suggesting the fiber made from the resin is rather adaptive for special applications.

The present invention aims at providing polyolefin resin-based non-woven fabric which substantially retains the features characteristic with the constituent resin represented by a polypropylene resin, such as high thermal resistance, high mechanical strength, etc., is excellent in softness, hand and touch feel, and is used particularly suitably as a sanitary material for the production of disposable diapers, sanitary napkins, etc., method for producing the same, and applications of the same.

### DISCLOSURE OF THE INVENTION

The present inventors studied hard to find a method by which to produce polyolefin resin-based non-woven fabric, particularly polypropylene resin-based non-woven fabric which retains the features characteristic with the constituent polyolefin resin such as good vapor permeability, waterproofness, softness, and high mechanical strength and thermal resistance, and which still presents with, in addition to spin easiness, a good use feeling based on hand and good touch feeling, the feature required for finished products prepared from the fabric. As a result, they found that the frictional property of the non-woven fabric has a grave influence on its use feeling, and achieved the invention based on this finding.

Briefly, the present invention includes following constitutive elements.
(1) Polyolefin resin-based non-woven fabric, which contains a fatty acid amide compound, and whose static frictional coefficient is 0.1 to 0.4.
(2) Polyolefin resin-based non-woven fabric as described in the paragraph (1) wherein the above fatty acid amide compound is erucamide and its content is 0.05 to 1.0 wt.%.
(3) Polyolefin resin-based non-woven fabric as described in (1) or (2) wherein the fiber of the fabric has a diameter of 1 - 50 µm.
(4) Polyolefin resin-based non-woven fabric cited in any one of the paragraphs (1) to (3) whose mass per unit area is 5 to 200 g/m².
(5) Polyolefin resin-based non-woven fabric cited in any one of the paragraphs (1) to (4) which is spunbonded non-woven fabric.
(6) Polyolefin resin-based non-woven fabric cited in any one of the paragraphs (1) to (5) wherein the polyolefin resin is a polypropylene resin.
(7) Polyolefin resin-based non-woven fabric cited in the paragraph (6) wherein the polypropylene resin is crystallizable and its isotactic pentad fraction is 88 to 95 mol.%.
(8) Polyolefin resin-based non-woven fabric cited in the paragraph (6) or (7) wherein the melt index (MI) of the polypropylene resin is 5 to 200 g/10 min.
(9) Multi-layered non-woven fabric comprising, at least in one surface, polyolefin resin-based non-woven fabric as cited in any one of the paragraphs (1) to (8).
(10) Multi-layered non-woven fabric cited in the paragraph (9) which has a three layered structure comprising a spunbonded non-woven layer/meltblown non-woven layer/spunbonded non-woven layer, and in which the mass per unit area of each non-woven layer is 7 to 25 g/m².
(11) Method for producing polyolefin resin-based non-woven fabric cited in any one of the paragraphs (1) to (10) comprising submitting non-woven fabric made of fiber obtained by melting a polyolefin resin containing a fatty acid amide compound and spinning it into fiber, to an aging treatment at 30 to 60°c for 5 to 50 hours.
(12) Sanitary materials made from polyolefin resin-based non-woven fabric cited in any one of the paragraphs (1) to (10).
(13) Sanitary materials cited in the paragraph (12) wherein the polyolefin resin is a polypropylene resin which is crystallizable, and whose isotactic pentad fraction is 88 to 95 mol.%.
(14) Sanitary materials cited in the paragraph (12) or (13) wherein the polyolefin resin is a polypropylene resin whose melt index (MI) is 30 - 80 g/10 min.
(15) Sanitary materials cited in any one of the paragraphs (12) to (14) which are used for the production of disposable diapers, sanitary napkins or incontinence pads.
(16) Sanitary articles made of a sanitary material cited in any one of the paragraphs (12) to (14).
(17) Sanitary articles cited in the paragraph (16) which are used for the production of disposable diapers, sanitary napkins or incontinence pads.

### BEST MODE FOR CARRYING OUT THE INVENTION

The present invention will be described in detail below.

The polyolefin resin-based non-woven fabric of this invention is characterized by containing a fatty acid amide compound, and by having a static friction coefficient of 0.1 to 0.4. The fatty acid amide compound is not limited to any specific one but may include any compound selected from the fatty acid amide compounds cited below, but preferably includes erucamide whose content is in the range of 0.05 to 1.0 wt.%, preferably 0.1 to 0.5 wt.%, or most preferably 0.2 to 0.4 wt.%.

The static friction coefficient of non-woven fabric can be determined in accordance with ASTM-D1894. Specifically, the measurement was performed under following conditions:
Tester of static friction coefficient: one provided by Toyo Seiki Manufacturing Co. (AN type);
Supportive plate: iron plate with a size of 63.6 mm x 102.2 mm x 19.4 mm (height), and weighing 8.87N; and
Gradient angle developing velocity: 2.7 degree/sec.

For non-woven fabric to be tested, two test pieces were prepared; the test surfaces were placed one over the other; the gradient angle was increased until the overlying piece starts to slide; and the sliding angle (?) was determined, to give tan ? which is used as the static friction coefficient of the test fabric. The lower the coefficient, the readier the test fabric is to slide.

The polyolefin resin-based non-woven fabric of this invention is not limited to any specific ones, but may include any such non-woven fabric obtained by a variety of manufacturing methods including the publicly known non-woven fabric such as spunbonded non-woven fabric, spunlace non-woven fabric, hot air-through bonding carded non-woven fabric, thermal embossing carded non-woven fabric, meltblown non-woven fabric, etc. Among them, what is preferred on account of mechanical strength and productivity may include spunbonded non-woven fabric based on long fibers, meltblown non-woven fabric adapted for a special function, and multilayered non-woven fabric comprising layers made of the above fabric.

However, the polyolefin resin-based non-woven fabric of this invention should have following features: it has a static friction coefficient in the range of 0.1 to 0.4 as described above, and thus, even if it has a multilayered structure, at least one exposed surface of the fabric has a static frictional coefficient of 0.1 to 0.4. If the polyolefin resin-based non-woven fabric of this invention has a multilayered structure, the other non-woven fabric used for concomitant layers may be made of a polyamide resin or a polyester resin, instead of a polyolefin resin.

The polypropylene resin used for the production of the polyolefin resin-based non-woven fabric of this invention is not limited to any specific ones, but may include homopolymers of propylene, or copolymers of propylene with at least one monomer selected from the a-olefins comprising ethylene, butene-1, 4-methyl-pentene-1, hexene-1, octene-1, etc. These polypropylene resins present with widely different crystallizing activities, molecular weights, and molecular weight distributions depending on the catalysts selected, and given polymerization conditions, and an appropriate one must be chosen from among them in accordance with the properties required from the target fabric.

In terms of the crystallizing activity, the resin used in this invention should be selected from those that have an isotactic pentad fraction in the range of 88 to 95 mol.%, preferably 89 to 93 mol.%, as long as the resin is used for the production of sanitary materials suitable for disposable diapers. The isotactic pentad fraction (IPF) is as defined in Macromolecules 28(16): 5403 (1995), that is, the fraction of isotactic species per pentad of a test polypropylene chain which is determined by radioactive carbon isotope-based nuclear magnetic resonance (¹³C-NMR).

In terms of the melt index (MI), the resin used in this invention should be selected from those whose MI is in the range of 5 to 200g/10 min, preferably 10 to 100g/10 min, when determined according to JIS K 7210 (temperature set at 230°c and load at 21.18N). Particularly, the resin whose MI is in the range of 30 to 80g/10 min is preferred, when it is used for the production of sanitary materials.

Next, the preferred polyethylene resin may include homopolymers of ethylene, and copolymers of ethylene with an a-olefin compound(s) with 3 to 10 carbon atoms, such as propylene, butene-1, 4-methyl-pentene-1, hexene-1, octene-1, etc. Particularly, copolymers of ethylene with an a-olefin monomer as described above having a density of 880 to 960 kg/m³, preferably 900 to 950 kg/m³, a melting point of 100 to 140°c, preferably 110 to 130°c, and an MI of 5 to 60g/10 min, preferably 10 to 50g/10 min (MI being determined according to JIS K 7210 where the temperature is set at 190°c, and load at 21.18N) are preferred on account of their melting point and mechanical strength.

The above polypropylene resin or polyethylene resin may occur as copolymers of two or more kinds of monomers. Or, they may occur as a resin composition to which is added another ethylene resin, another propylene resin or a thermoplastic elastomer, as long as the addition is kept at 50 wt.% or lower.

The polyolefin resin-based non-woven fabric of this invention must contain, without fail, a fatty acid amide compound. The fatty acid amide compound used in this invention may include fatty acid monoamide compounds, fatty acid diamide compounds, saturated monoamide compounds, unsaturated diamide compounds, etc. Specifically, it may include lauramide, myristamide, palmitamide, stearamide, behenamide, oleinamide, erucamide, montamide, N, N'-methylene-bis-lauramide, N, N'-methylene-bis myristamide, N, N'-methylene-bis palmitamide, N, N'-methylene-bis-behenamide, N, N'-methylene-bis-oleinamide, N, N'-methylene-bis-erucamide, N, N'-ethylene-bis-oleinamide, N, N'-ethylene-bis-erucamide, etc. Two or more of them may be combined.

Among these fatty acid amide compounds, erucamide or an unsaturated fatty acid monoamide compound is preferably used. The reason is ascribed to the facts that the non-woven fabric containing such a compound is safely protected against degraded spinning easiness which would otherwise result from the deposition of fatty acid amide on the fiber surface during the melt being spun into fiber, and that the non-woven fabric containing such a compound will have a more lowered static friction coefficient as a result of the aging as will be described later. The content of the fatty acid amide compound with respect to the amount of the polyolefin resin is in the range of 0.05 to 1 wt.%, preferably 0.1 to 0.5 wt.%. The content in question should be determined with due consideration being paid to general conditions including the species of the fatty acid amide compound, the species of the concomitant polyolefin resin as well as the characteristics of the latter including its crystallizing activity, MI, etc., and the properties required from the target fabric, and the aging.

If, for example, erucamide is used in conjunction with a propylene homopolymer whose isotactic pentad fraction is about 90 mol.%, its content should be in the range of 0.1 to 0.5 wt.%, most preferably 0.2 to 0.4 wt.%. In such a case, if the content of erucamide is below 0.2 wt.%, it will be difficult for the fabric to have a static friction coefficient of 0.1 to 0.4, depending on the aging condition. On the contrary, if the content of erucamide is over 0.4 wt.%, its deposition on the fiber surface will become considerable, which may result in the development of whiteness on the fiber surface, degrading its appearance, or cause the lowered thermal adhesiveness of fibers.

The polyolefin resin-based non-woven fabric of this invention may contain a publicly known additive, so as to be adaptive for a given application or to be provided with a certain desired property. Such publicly known additives may include neutralizers such as calcium stearate, hydrotalcite, etc.; antioxidants such as phenol-, phosphate-, sulfate-based compounds; thermal stabilizers; nucleating agents; UV absorbers; photic stabilizers; anti-statistics; flame-retarders; pigments; dyes; and inorganic powders such as silica, talc, calcium carbonate, calcium oxide, magnesium oxide, etc.

The polyolefin resin-based non-woven fabric of this invention may exist as primary non-woven fabric which is obtained by adding, in addition to an additive which is introduced as needed, a specified amount of fatty acid amide to a polyolefin resin, dryblending the mixture, melting the yield, spinning the melt into fiber, and combining the fibers. The method by which the melt is turned into fibers and the fibers combined into fabric may include spunbonding, meltblowing, spunlacing, carding with hot air-through bonding, carding with thermal embossing, etc. Among them, the spunbonding and the meltblowing, particularly the spunbonding may be profitably employed because the fabric produced by the method presents with an excellent mechanical strength, functionality and productivity.

The spunbonded non-woven fabric may be obtained as primary non-woven fabric, for example, by the following publicly known method: a polyolefin resin having a composition as mentioned above is melted in an extrusion molding device, and extruded therefrom; spinning extruded from a spinneret is spun; the spun fibers are pulled with an air current-based collecting device such as an air sucker, while the collected fibers being opened as needed; the fibers are collected on a web catcher such as an air current-based net conveyer; the fibers are partially bonded with a heating means based on hot blowing or on a heating roll, and the resulting fabric is wound around a cylinder.

The above polyolefin resin-based non-woven fabric is normally made of fibers composed of a polyolefin homopolymer, but the fabric may be made of bicomponent fiber, as long as 50% or more of the surface of each fiber is accounted for by a single polyolefin resin.

The non-woven fabric made of bicomponent fiber may include the one in which each fiber has a skin-core structure in which the skin is made of a polyolefin resin and the core of a resin other than the polyolefin resin such as a polyamide resin or a polyester resin having a higher melting point than does the polyolefin resin, or the one with a side-by-side structure in which fibers made of an polyolefin resin and fibers made of another resin are placed side by side in such a way as to make the former fibers account for 50% or more wt.% of the fabric. Needless to say, two different polyolefin resins may be utilized for the formation of the above two kinds of fabric based on complicated fibers: they may form the skin and the core of each fiber for the former fabric, while they may form one and the other kinds of fibers for the latter fabric.

As regards the diameter of the fibers constituting the polyolefin resin-based non-woven fabric of this invention, it is not limited to any specific value. However, it may be in the range of 1 - 50 µm, preferably 5 - 40 µm. Particularly, when the fabric is used as a sanitary material, the diameter in question is most preferably in the range of 10 to 25 µm. The mass per unit area of the constitutive fiber is not limited to any specific value, but usually it is in the range of 5 to 200 g/m², preferably 10 to 100 g/m². If the fabric is used as a sanitary material for the production of disposable diapers, and produced by spin bonding, and consists of a single layer, its mass per unit area is preferably in the range of 10 to 30 g/m², most preferably 18 to 25 g/m². If the fabric has a three-layered structure comprising spunbonded fabric/meltblown fabric/spunbonded fabric layers, its mass per unit area is preferably in the range of 7 to 25 g/m², particularly preferably 12 to 17 g/m².

The primary non-woven fabric produced as above, however, does not give a static friction coefficient as specified for the fabric of this invention unless otherwise treated, even though it presents with excellent spinning easiness. The above primary non-woven fabric will give a static friction coefficient falling within the range as specified for the polyolefin resin-based non-woven fabric of this invention, if it receives an aging treatment under heating. Generally, aging has not been conducted for this purpose.

The conditions for the aging treatment vary according to the species of the polyolefin resin, its characteristics such as the crystallizing activity, density and melting point, and the species of the coexistent fatty acid amide compound, its melting point, and solubility to the polyolefin resin. The conditions in question should be determined with due consideration being paid to the characteristics of the polyolefin resin serving as a material of the fabric, the characteristics of the coexistent fatty acid amide, the likeliness of the fabric to give a static friction coefficient of 0.1 to 0.4, and the hand and touch feeling required for the final product. For details, the conditions must be determined experimentally.

The method provided by this invention comprises submitting non-woven fabric which is obtained after having melted a polyolefin resin containing a fatty acid amide compound and spun it into fiber, to an aging treatment at 30 to 60°c for 5 to 50 hours. However, the method for producing the polyolefin resin-based non-woven fabric of this invention is not limited to the ranges given above.

Take, for illustration, a case where, to produce non-woven fabric of this invention, fiber is utilized which is made of a propylene homopolymer, has an isotactic pentad fraction of about 90 mol.%, and contains 0.3 wt.% erucamide. Then, the preferred conditions for aging are as follows.

If aging occurs at 40°c, the aging time is about 5 to 50 hours, preferably eight to 12 hours. Conversely, if the aging time is 24 hours, the aging temperature should be 32 to 50°c, preferably 33 to 40°c. If the aging condition is milder than above, the lowering speed of the static friction coefficient of the fabric may become so low as to interfere with the productivity. On the contrary, if the aging condition is sterner than above, the fabric may give a higher static friction coefficient than specified, which is undesirable.

The aging treatment normally occurs in an aging chamber where fabric pieces are rolled around cylindrical cores, the pieces are neatly arranged with the cores being placed side by side, and hot air is allowed to circulate round the fabric pieces. During aging, even if the fabric piece is wound around a core, it has such a sufficient air permeability as to permit its entire mass to be essentially uniformly subject to aging.

The polyolefin resin-based non-woven fabric of this invention, because of its lowered static friction coefficient due to the aging, is greatly improved in its hand and touch feeling, while maintaining the features characteristic with the non-woven fabric based on the polyolefin resin fiber. Accordingly, it may be used for the production of various sanitary materials as well as of clothing materials.

The touch feeling is only determined by the features of the surface of the fibers constituting the non-woven fabric. Thus, it is possible to improve the characteristics of the original fabric such as its strength, vapor permeability, dust shielding activity, thermal adhesiveness, etc., by adding, as appropriate, common polyolefin resin-based fabric, thermoplastic resin-based fabric, etc., or other sheet materials such as a vapor permeable film, water proof film, water repellent film, etc., neither of which, however, contains fatty acid amide compound, so as to allow the resulting fabric to have a multilayered structure, as long as the addition does not affect the surface characteristics of the fibers of the original fabric.

If such addition is made, the layering should be normally conducted in such a manner as to make the polyolefin resin-based non-woven fabric having undergone aging be exposed at least on one side of the layered fabric. However, in certain cases, it is possible to overlap a layer of the polyolefin resin-based non-woven fabric with another layer of different fabric, and then to submit the resulting layered fabric complex to aging. The different fabric suitable to be incorporated in such a layered structure may include spunbonded non-woven fabric, meltblown non-woven fabric, and staple non-woven fabric. For example, take non-woven fabric having a multilayered structure obtained by overlapping a meltblown non-woven fabric layer over a spunbonded non-woven fabric layer, and then by placing another spunbonded non-woven fabric layer over the foregoing assembly. To produce such non-woven fabric, it is recommended to firstly prepare multilayered fabric of which at least one spun bound non-woven fabric layer made of a polyolefin resin is allowed to contain fatty acid amide, and then to submit this multilayered fabric to aging.

Besides non-woven fabric made of a polyolefin resin, the non-woven fabric according to this invention may include a fabric layer made of, for example, a polyester resin or a polyamide resin, particularly the one having a melting point of 150°c or higher, more preferably 150 to 300°c. The preferred polyester resin may include homopolymers such as polyethylenetelephthalate, polybutylenetelephthalate, polytrimethylenetelephthalate, polynaphtalenetelephthalate, etc., and the copolymers of the above constitutive monomers with other monomers, and the combinations of such copolymers.

The preferred polyamide resin may include, to mention a few for illustration, nylon 6 (polycaprolactamide), nylon 6,6 (polyhexamethyleneadipoamide), nylon 6,10 (polyhexamethylenecebacamide), nylon 11 (polyundecanamide), nylon 7 (poly-?-aminoheptanate), nylon 9 (poly-? -aminononate), nylon 12 (polylaurinamide), etc. Among them, nylon 6 and nylon 6,6 are preferred.

For laminating non-woven fabric layers to produce non-woven fabric with a multilayered structure, various methods including thermal bonding or adhesive bonding may be applicable. However, emboss rolling under heating is particularly recommendable because of its simplicity and low cost. The emboss rolling under heating may occur with a publicly known laminating device based on the combination of an emboss roll and a flat roll. The emboss rolling may occur by any patterned rolling including, for example, grid patterned rolling by which adhesive continuous lines form a grid pattern, independent pattern rolling, or desired pattern rolling. The patterned bonded area may account for 5 to 40% of the total area.

The polyolefin resin-based non-woven fabric of this invention may have a multilayered structure comprising, in addition to other non-woven fabric layers, a layer such as a vapor permeable layer (film), water impermeable layer (film) or water repellent layer (film). For the production of such non-woven fabric, extrusion lamination, emboss rolling under heating or dry lamination may be employed.

The condition suitable for proper lamination by emboss rolling under heating may vary depending on the melting points of the polyolefin resin-based fabric layer and of the other concomitant non-woven fabric layers, and on which one among plural films is exposed to embossing, and should be determined with due consideration being paid to those involved factors. Thus, the emboss pattern and relative emboss area, and the temperature and pressure under which embossing occurs should be determined as appropriate according to the diameter of the fibers constituting individual fabric layers, the thickness, mass per unit area, air permeability and processing speed of individual fabric layers, and, in addition, the melting point and thickness of concomitant films.

The polyolefin resin-based non-woven fabric of this invention, method for producing the same, and examples produced by the method will be described in detail below. However, it should be understood that the present invention is not restricted in any way in its scope by those examples.

### [Example 1]

A crystallizable polypropylene resin (IPF: 91 mol.%, MI: 60 g/10 min, melting point: 160°c) 100 parts by weight, 0.035 part by weight of a phenol antioxidant (Ilganox 1010, Chiba Special Chemicals Co.), 0.035 part by weight of a phosphate antioxidant (Sandstab P-EPQ, Sand Co.), 0.035 part by weight of a neutralizer (Kyodo Pharmaceutical Co.), 0.025 part by weight of calcium stearate, and erucamide having a weight as specified in Table 1 were dry-blended with a super mixer; the mixture was melted and kneaded at 220°c in an extrusion molding device with 65mmF; the melt was extruded through spinneret to be spun into fiber. Arrays of spinneret are arranged as follows: the diameter of each spinneret is 0.3 mm, and the spinnerets number 200 in the width direction while they number 15 in the extrusion direction. The spun fiber had an average diameter of 18 µm (ten fibers were arbitrarily chosen, and placed under a microscope with a scale for measurement).

A group of fibers were introduced into an air sucker to be stretched; the fibers were then collected on a belt equipped with an aspirator to be inserted between a pair of rolls for heat embossing (an embossed roll kept at 140°c and a flat roll kept at 140°c) to be partially bonded; and then the resulting sheet was wound around a paper cylinder to produce spunbonded non-woven fabric. The non-woven fabric wound around the paper cylinder was submitted to aging, which consisted of keeping the fabric at a specified temperature for a specified period, to produce the polyolefin resin-based non-woven fabric of this invention. Following the above procedures, a number of non-woven fabric samples were obtained with the content of erucamide slightly varied, and they were evaluated for their mass per unit area, spin easiness and other involved characteristics. The results are shown in Table 1.

For a given non-woven fabric sample, its spin easiness and physical characteristics were evaluated as follows.
(1) Spin easiness
   The non-woven fabric sample was evaluated as excellent (? ), good (? ) and normal (? ), according to the behavior of the fiber during spinning, that is, whether it was not broken, whether it did not show any fluctuation, and whether it stably yielded to spinning.
(2) Static friction coefficient
   The static friction coefficient of the non-woven fabric sample was determined according to ASTM-D1894. The detail of the method was described above.
(3) Tensile strength
   It was measured according to JIS L 1906.
(4) Stiffness
   It was determined according to JIS L 1096 (45º cantilever method).
(5) Touch feeling/hand
   Twenty testers touched the test fabric samples, and evaluated the samples according to the touch feel they gave as excellent (ⓞ), good (○) and normal (Δ).

The results are shown in Table 1.

From Table 1, it is apparent that the fabric samples (2), (3) and (4) present with static friction coefficients which fall within the range specified for the non-woven fabric of this invention, and thus they are more suitable as a sanitary material for disposable diapers, as compared with the fabric samples (1) and (5).

**Table 1**

| | Example 1 | | | | |
|---|---|---|---|---|---|
| | (1) | (2) | (3) | (4) | (5) |
| Addition amount of erucamide (weight part) | 0.1 | 0.2 | 0.3 | 0.4 | - |
| Mass per unit area (g/m²) | 20 | 20 | 20 | 20 | 20 |
| Spin easiness | ⓞ | ⓞ | ⓞ | ⓞ | ⓞ |
| Aging temperature (°c) | 40 | 40 | 40 | 40 | 40 |
| Aging time (hours) | 24 | 24 | 24 | 24 | 24 |
| Static friction coefficient | 0.45 | 0.28 | 0.19 | 0.18 | 0.65 |
| Stiffness (cm) | 6.0 | 5.9 | 5.3 | 5.3 | 6.4 |
| Touch feeling | Δ | ○ | ⓞ | ⓞ | Δ |
| Hand | Δ | ○ | ⓞ | ⓞ | Δ |

### [Example 2]

Non-woven fabric samples were obtained by the same method as used in Example 1, except that the content of erucamide was made constant, the aging temperature was kept constant and the aging time was varied. The non-woven fabric samples were evaluated for their mass per unit area, spin easiness, and other involved characteristics. The results are shown in Table 2.

From Table 2 it is apparent that the fabric samples (2), (3) and (4), particularly samples (3) and (4) present with static friction coefficients which fall within the range specified for the non-woven fabric of this invention, and thus they are more suitable as a sanitary material for disposable diapers, as compared with the fabric sample (1).

**Table 2**

| | Example 2 | | | |
|---|---|---|---|---|
| | (1) | (2) | (3) | (4) |
| Addition amount of erucamide (weight part) | 0.3 | 0.3 | 0.3 | 0.3 |
| Mass per unit area (g/m²) | 20 | 20 | 20 | 20 |
| Spin easiness | ⓞ | ⓞ | ⓞ | ⓞ |
| Aging temperature (°c) | 40 | 40 | 40 | 40 |
| Aging time (hours) | 0 | 6 | 12 | 24 |
| Static friction coefficient | 0.55 | 0.35 | 0.22 | 0.19 |
| Stiffness (cm) | 6.1 | 5.9 | 5.8 | 5.3 |
| Touch feeling | Δ | Δ○ | ○ | ⓞ |
| Hand | Δ | Δ○ | ⓞ | ⓞ |

### [Example 3]

Non-woven fabric samples were obtained by the same method as used in Example 1, except that the content of erucamide was made constant, the aging time was kept constant and the aging temperature was varied. The non-woven fabric samples were evaluated for their mass per unit area, spin easiness, and other involved characteristics. The results are shown in Table 3.

From Table 3 it is apparent that the fabric samples (3), (4), and (5) present with static friction coefficients which fall within the range specified for the non-woven fabric of this invention, and thus they are more suitable as a sanitary material for diapers, as compared with the fabric samples (1) and (2). It is observed by inspecting the results of the sample (5) that the fabric sample exposed to a higher temperature during aging tends to develop a higher static friction coefficient.

**Table 3**

| | Example 3 | | | | |
|---|---|---|---|---|---|
| | (1) | (2) | (3) | (4) | (5) |
| Addition amount of erucamide (weight part) | 0.3 | 0.3 | 0.3 | 0.3 | 0.3 |
| Mass per unit area (g/m²) | 20 | 20 | 20 | 20 | 20 |
| Spin easiness | ⓞ | ⓞ | ⓞ | ⓞ | ⓞ |
| Aging temperature (°c) | 23 | 28 | 35 | 40 | 45 |
| Aging time (hours) | 24 | 24 | 24 | 24 | 24 |
| Static friction coefficient | 0.50 | 0.45 | 0.19 | 0.18 | 0.25 |
| Stiffness (cm) | 6.1 | 6.0 | 5.3 | 5.3 | 5.8 |
| Touch feeling | Δ | Δ | ⓞ | ⓞ | ○ |
| Hand | Δ | Δ | ⓞ | ⓞ | ○ |

### [Example 4]

In stead of the device for producing spunbonded non-woven fabric used in Example 1, a multi-stage device was employed, and erucamide containing polypropylene used in Example 1 and erucamide-free polypropylene were introduced as materials to produce non-woven fabric having a two layered structure each layer having an mass per unit area of 10 g/m², or the density different from those of Examples 1 to 3. The non-woven fabric was submitted to aging, which consisted of keeping the fabric at 40°c for 24 hours. A number of non-woven fabric samples were obtained, and they were evaluated for their mass per unit area, spin easiness and other involved characteristics. The results are shown in Table 4.

From Table 4 it is apparent that the layered non-woven fabric also presents with excellent characteristics.

### [Example 5]

Three layered non-woven fabric was obtained by the same method as used in Example 4, except that the mass per unit area of each layer was varied to 7 g/m², and that a layer (mass per unit area being 3 g/m²) comprising meltblown non-woven fabric made of an erucamide free polypropylene resin was laid over the spunbonded non-woven fabric layer produced in the foregoing stage. The multi-layered non-woven fabric thus obtained was submitted to aging, which consisted of keeping the fabric at 40°c for 24 hours. A number of multi-layered non-woven fabric samples were obtained, and they were evaluated for their mass per unit area, spin easiness, and other involved characteristics. The results are shown in Table 4.

From Table 4 it is apparent that the non-woven fabric of this invention presents with excellent characteristics, even when it is modified so as to have a layered structure.

**Table 4**

| | | Example 4 | | Example 5 | |
|---|---|---|---|---|---|
| | | (1) | (2) | (1) | (2) |
| Addition amount of erucamide (weight part) | Layer A | 0.3 | 0.3 | 0.3 | 0.3 |
| | Layer B | no | no | 0 | 0 |
| | Layer C | 0.3 | 0 | 0.3 | 0 |
| Mass per unit area (g/m²) | | 20 | 20 | 17 | 17 7 |
| Spin easiness | | ⓞ | ⓞ | ⓞ | ⓞ |
| Static friction Static friction | Surface A | 0.19 | 0.22 | 0.25 | 0.22 |
| coefficient | Surface B | 0.20 | 0.57 | 0.27 | 0.60 |
| Stiffness (cm) | | 5.3 | 5.5 | 5.5 | 5.8 |
| Touch feeling | Surface A | ⓞ | ⓞ | ⓞ | ⓞ |
| | Surface B | ⓞ | Δ | ⓞ | Δ |
| Hand | Surface A | ⓞ | ⓞ | ⓞ | ⓞ |
| | Surface B | ⓞ | Δ | ⓞ | Δ |

### Industrial Applicability

This invention provides a method for producing polyolefin resin-based non-woven fabric excellent in hand and touch feeling. That fabric can be suitably used as sanitary articles such as disposable diapers, or as a material of such sanitary articles.

## Claims

1. Polyolefin resin-based non-woven fabric **characterized by** that it contains a fatty acid amide compound, and that its static friction coefficient is 0.1 to 0.4.

2. Polyolefin resin-based non-woven fabric as described in Claim 1 wherein the fatty acid amide compound is erucamide, and its content is 0.05 to 1.0 wt.%.

3. Polyolefin resin-based non-woven fabric as described in Claim 1 or 2 wherein the fiber constituting the non-woven fabric has a diameter of 1 to 50 µm.

4. Polyolefin resin-based non-woven fabric as described in any one of Claims 1 to 3 which has an mass per unit area of 5 to 200 g/m².

5. Polyolefin resin-based non-woven fabric as described in any one of Claims 1 to 4 which is spunbonded non-woven fabric.

6. Polyolefin resin-based non-woven fabric as described in any one of Claims 1 to 5 wherein the polyolefin resin is a polypropylene resin.

7. Polyolefin resin-based non-woven fabric as described in Claim 6 wherein the polypropylene resin is crystallizable, and has an isotactic pentad fraction of 88 to 95 mol.%.

8. Polyolefin resin-based non-woven fabric as described in Claim 6 or 7 wherein the melt index (MI) of the polypropylene resin is 5 to 200 g/10 min.

9. Multi-layered non-woven fabric comprising polyolefin resin-based non-woven fabric as described in any one of Claims 1 to 8 at least on one surface.

10. Multi-layered non-woven fabric as described in Claim 9 wherein the fabric has a three layered structure comprising a spunbonded non-woven fabric layer/meltblown non-woven fabric layer/spunbonded non-woven fabric layer, and those non-woven fabric layers have an mass per unit area of 7 to 25 g/m².

11. A method for producing polyolefin resin-based non-woven fabric as described in any one of Claims 1 to 10 comprising subjecting non-woven fabric obtained by melting a polyolefin resin containing a fatty acid amide compound and by spinning the melt into fiber, to an aging treatment at 30 to 60°c for 5 to 50 hours.

12. A sanitary material comprising polyolefin resin-based non-woven fabric as described in any one of Claims 1 to 10.

13. A sanitary material as described in Claim 12 wherein the polyolefin resin is a polypropylene resin which is crystallizable and has an isotactic pentad fraction of 88 to 95 mol.%.

14. A sanitary material as described in Claim 12 or 13 wherein the polyolefin resin is a polypropylene resin which has a melt index (MI) of 30 to 80 g/10 min.

15. A sanitary material as described in any one of Claims 12 to 14 which is applicable to a disposable diaper, sanitary napkin or incontinence pad.

16. A sanitary article made from a sanitary material as described in any one of Claims 12 to 14.

17. A sanitary article as described in Claim 16 which is applicable to a disposable diaper, sanitary napkin or incontinence pad.
